# EUROPEAN PATENT APPLICATION

(11) **EP 2 039 624 A1**
(43) Date of publication of application: **25.03.2009**
(21) Application number: 08007443.8
(22) Date of filing: 16.04.2008
(51) Int. Cl.: B65D 83/14, B65D 83/16, A61L 9/14, B67D 5/33, E03D 9/00

(54) **Spraying device and method of using same**

(30) Priority: 21.09.2007 GB 0718457
(71) Applicant: Reckitt Benckiser (UK) Limited, Slough, Berkshire SL1 3UH (GB)
(72) Inventor: Butler, Martin, Hull, HU8 7DS (GB); Walsh, Steve, Hull, HU8 7DS (GB)
(74) Representative: Carlin, Robert George

(57) **Abstract**

A spraying device (10) is described, the device comprising a housing (12) adapted to receive a refill (16) of fluid therein and having an aperture (14) suitable for permitting, in use, the spraying of a fluid therethrough, the device further comprising actuation means (24) configured for periodic actuation of the refill, wherein the device is provided with detection means (38) configured to distinguish between at least one area of lower magnetism and at least one area of relatively higher magnetism on a spray head of said refill or within the area normally occupied by said spray head. Methods of operation of such a spraying device is also described.

## Description

### Field of the Invention

The present invention relates to a device for spraying a fluid and particularly, but not exclusively to a device for spraying fluids such as fragrances, deodorizing fluids and/or a pest control materials or the like, The present invention also relates to a method of using such a device.

### Background

Prior art devices for spraying fragrances, deodorising agents and sanitising fluids into a room generally consist of a device containing a removable source of fluid. With such an arrangement once the source of fluid has been completely exhausted, the source can be replaced rather than replacing the entire device. Typically such sources come in many forms, including containers, bottles, cans and cartridges (generically, all such containers, bottles, cans and cartridges hereinafter will be referred to as "refills"). Such refills can be pump sprays or aerosols, including metered and non-metered versions thereof.

Known prior art devices typically comprise a housing having an opening through which the fluid is sprayed. A part of the housing is movable/removable to permit a refill to be introduced and subsequently removed from the interior of the device. The device further comprises a mechanically actuated arm or the like therein which is adapted to periodically activate in order to press down on a spray head secured to the refill, resulting in fluid passing from the body of the refill, through the spray head and out of the opening into the surrounding environment.
The shape of a refill is typically standardized to a certain degree, as such, spraying devices may unwittingly provide an opportunity to facilitate vandalism or dangerous behaviour. Specifically, vandals or the like may seek to intentionally insert a dangerous source of sprayable fluid into a spraying device. For instance, where the device is for spraying a fragrance and the refill is an aerosol of a standard size, one form of vandalism and/or intentional abuse could be the insertion of a paint aerosol into the device. The resulting damage from such abuse could be substantial. Such abuse would likely be associated with a significant health and safety risk. Clearly it would be desirable from a user's perspective to be protected from such dangerous behaviour and/or acts of vandalism.

It is an object of the present invention to address the above mentioned concerns and disadvantages.

### Summary of Invention

According to a first aspect of the present invention there is provided therefore a spraying device comprising a housing adapted to receive a refill of fluid therein and having an aperture suitable for permitting, in use, the spraying of a fluid therethrough, the device further comprising actuation means configured for periodic actuation of the refill, wherein the device is provided with detection means configured to distinguish between at least one area of lower magnetism and at least one area of relatively higher magnetism on a spray head of said refill or within the area normally occupied by said spray head.

For the avoidance of doubt, the term "lower magnetism" is used herein as a relative term with reference to the term "higher magnetism", "lower" may include areas of zero or near zero magnetism.

In the context of the present invention a refill is understood to mean a container of fluid comprising a body, which may be elongate, in the form of a reservoir for a fluid, an outlet stem located in an upper portion of the body and a spray head that attached to the free end of the outlet stem to be preferably located at the uppermost part of the refill. Therefore, the area normally occupied by the spray head is understood to mean the area toward the uppermost part of the refill within the context of the present invention.

According to a second aspect of the present invention there is provided therefore a spraying device comprising a housing adapted to receive a refill of fluid therein and having an aperture suitable for permitting, in use, the spraying of a fluid therethrough, the device further comprising actuation means configured for periodic actuation of the refill, wherein the device is provided with detection means configured to distinguish between at least one area of lower magnetism and at least one area of relatively higher magnetism on a spray head of said refill or within the area normally occupied by said spray head, and wherein said detection means operate during movement of the spray head.

According to a third aspect of the present invention there is provided therefore a spraying device comprising a housing adapted to receive a refill of fluid therein and having an aperture suitable for permitting, in use, the spraying of a fluid therethrough, the device further comprising actuation means configured for periodic actuation of the refill, wherein the device is provided with detection means configured to distinguish between one area of lower magnetism and one area of relatively higher magnetism on a spray head of said refill.

According to a fourth aspect of the present invention there is provided therefore a spraying device comprising a housing adapted to receive a refill of fluid therein and having an aperture suitable for permitting, in use, the spraying of a fluid therethrough, the device further comprising actuation means configured for periodic actuation of the refill, wherein the device is provided with detection means configured to distinguish between one area of lower magnetism and one area of relatively higher magnetism on a spray head of said refill or within the area normally occupied by said spray head, and wherein said detection means operate during movement of the spray head.

Advantageously the device according to the present invention may be able to detect when a user attempts to use the device with a potentially dangerous refill and, further, the device is preferably configured to deny the actuation means from activating and/or deny subsequent activation whilst such a refill is loaded in the device to prevent and/or limit any damage that could result from actuating the refill.

A further advantage of the devices of the present invention is that they may be able to provide an improved end of life indication. In use, when a device according to the present detects the at least one area of lower magnetism and at least one area of relatively higher magnetism on a spray head of the refill loaded therein, or within the area normally occupied by the spray head when loaded in the device, that may trigger a counting mechanism. The counting mechanism may be calibrated to allow a pre-determined number of actuations of the refill which corresponds to the quantity of fluid stored in a refill. The counting mechanism may be operable in use, and after the pre-determined number of actuations has been reached, to prevent the device from causing further actuations of that refill until a user replaces the refill and/or resets the device. The device may automatically reset every time a refill is loaded into the device. This end of life indication may be advantageous as the power consumption of the device will be minimised, this would be particularly advantageous where the device is battery powered or the like.

The counting mechanism may be linked to an indicator that is adapted to communicate to the user of the device that a used or spent refill needs to be replaced.

Preferably the pre-defined n umber of actuations is calculated to correspond to the quantity of fluid in the refill.

Preferably the detection means are in direct communication with the actuation means such that, in use, the detection means may instruct the actuation means not to activate.

Alternatively a control means may be provided that is in direct communication with both the detection means and the actuation means, said control means being operative to receive an input from the detection means and being operative to instruct the actuation means to activate or not depending on the received input

The control means may be provided in the form of a micro processor, a circuit provided on a PCB or in the form of other convenient component(s).

Preferably the actuation means are operable to cause activation of the refill spray head by imparting a substantially downward force on the spray head. Said substantially downward force is preferably sufficient to cause the spray head to move in a substantially downward direction to cause the spraying of a quantity of fluid from the body of the refill, through the spray head and out of the opening in the housing into the surrounding environment Once a quantity of fluid has been sprayed, the device may be configured such that the refill's inherent resilience and/or internal pressure is capable of applying a substantially upward force on the spray head sufficient to return the actuation means to its starting position without the need for power to be applied to said means.

The detection means of the present invention is adapted, in use, to distinguish between areas of differing magentism on a spray head of a refill or within the area normally occupied by such a spray head. Preferably the detection means is capable of distinguishing as mentioned by interrogating the refill. The refill may be interrogated by the detection means such that the amount of magnetism can be attributed specifically or approximately or generally to a section of the spray head or area normally occupied thereby. Such attribution may permit the detection means to directly, or in combination with a control unit, determine whether any areas of differing magentism are present on the spray head or in the area normally occupied thereby.

If there are no areas of differing magnetism on the spray head of the refill, or in the areas normally occupied thereby, there should be a substantially constant level of magnet property across the whole spray head. Therefore in an alternative arrangement, the interrogation of the refill by the detection means may permit the detection means to detect whether at least one differing level of magnetism is detected without attributing the detected level of magnetism to a specific section of the spray head or the area normally occupied thereby. Such a detection may permit the detection means to directly, or in combination with a control unit, determine whether any areas of differing magentism are present on the spray head or in the area normally occupied thereby.

The detection means of the device according to either the second and fourth aspects may be configured to only be operable during the movement of the spray head. This may be advantageous since this would permit the detection means on the device to have a fixed location with no moving parts, thus reducing the cost of the detection means and rendering them less likely to fail during the life of the device. Additionally, with such an arrangement the detection means may have a predefined field of view allowing a reputable refill manufacturer more defined parameters for ensuring the spray head is suitably detectable be the detection means.

Preferably the detection means according to either the second or fourth aspects may be confined to only be operable during the substantially upward movement of the spray head, i.e. the upward movement of the spray head after the downward movement caused by the actuation means. In this arrangement the inherent resilience and/or internal pressure of the refill may be sufficient to return the actuation means to its starting position without power being applied to said means. Such an arrangement may be particularly advantageous since the substantially upward movement of the spray head may be achieved without power being applied to the activation means, thus reducing or eliminating electrical nolse/interference produced by the device. The reduction in electrical noise/interference may improve the ability of the detection means to distinguish areas of differing magnetism on the spray head.

Alternatively or additionally the detection means may be configured to move in order to scan the spray head or the area normally occupied by the spray head. The detection may be movable in a substantially horizontal direction and/or a substantially vertical direction and/or in at least two directions. The detection means may be able to pivot about a single position to perform the scan. The detection means may be provided with a wide-angle collection means, to provide a wide field of view when performing the scan.

The detection means may be provided in the form of one or more sensors.

The detection means may have an effective range of up to 100mm, the effective range being the distance at which magnetic properties may be detected by the detection means. Preferably the detection means has an effective range of up to 50mm, and more preferably up to 20mm, and most preferably up to 5mm.

According to a fifth aspect of the present invention there is provided therefore a method of spraying a fluid from a device, the method comprising the steps of loading a refill in a spray device according to any of the first to fourth aspects of the present invention, placing the device in an operation mode, said mode being configured to cause activation of actuation means, said actuation means bearing against a spray head of the refill and causing the movement thereof to release a quantity of fluid from the refill, said released fluid being sprayed from the device through an aperture into the surrounding environment, wherein detection means provided in the device are operable in the operation mode to detect and distinguish between at least one area of lower magnetism and at least one area of relatively higher magnetism on a spray head of said refill or within the area normally occupied by said spray head.

The method of the fifth aspect of the present invention is operable to prevent the activation of the activation means if the detection means is unable to detect and distinguish between said at least two areas of differing magnetism.

According to a sixth aspect of the present invention there is provided therefore a method of spraying a fluid from a device, the method comprising the steps of loading a refill in a spray device according to any of the first to fourth aspects of the present invention, placing the device in an operation mode, said mode being configured to cause activation of actuation means, said actuation means bearing against a spray head of the refill and causing the movement thereof to release a quantity of fluid from the refill, said released fluid being sprayed from the device through an aperture into the surrounding environment, wherein detection means provided in the device are operable in! the operation mode to detect and distinguish between at least one area of lower magnetism and at least one area of relatively higher magnetism on a spray head of said refill or within the area normally occupied by said spray head during the activation of same by the actuation means.

Preferably the detection means according to the sixth aspect may be configured to only be operable during the movement of the spray head. Even more preferably, these detection means are confined to only be operable during the substantially upward movement of the spray head, i.e. the upward movement of the spray head after the downward movement caused by the actuation means.

The method of the sixth aspect of the present invention is operable to prevent further activation of the activation means if the detection means is unable to detect and distinguish between said two areas of differing magnetism during the initial activation by the actuation means.

Magnetic paints/ink/lacquers or the like may be used to provide the areas of magnetism. These areas may be provided by areas of the spray head having said magnetic paints/ink/lacquers or the like applied thereto to contrast with areas on the spray head having no such application. Alternatively, the areas may be provided by the application of at least two distinct magnetic paints/ink/lacquers or the like to the spray head, wherein said distinct magnetic paints/ink/lacquers or the like have differing magnetic properties which are distinguishable from each other.

The areas of differing magnetism may be applied or incorporated on the spray head by any suitable means. Particularly preferred methods include the initial application of the areas of differing magnetism to a label which is subsequently attached to the spray head. Alternatively, the areas of differing magnetism may be printed, engraved and/or applied directly on to the spray head. As a further alternative, the spray head could be manufactured from two or more component parts, wherein at least two of said parts are provided with external areas thereof having distinguishable magnetic properties with respect to each other.

Typically the spray head is made via injection molding as one-piece component In an alternative arrangement the outlet stem could be provided with magnetic properties and the spray head may be provided with a corresponding window in registration with the outlet stem to permit the magnetic properties of the outlet stem to be detected by the detection means to provide the areas of differing magnetism.

The spray head of a refill typically has a generally L-shaped profile, wherein the shorter part of the L-shape engages with the free end of the outlet stem. Preferably the spray head of the refill for use with the device of the present invention is generally L-shaped. In an alternative arrangement, the spray head could be provided with a cutaway. Such a cutaway may be located within the area of the spray head that is detectable by the detection means such that the detection means' scan of the spray head detects the difference in the magnetism of the spray head against the absence of any magnetism in the cutaway. Alternatively the spray head may be shaped into a non-typical shape (i.e. not an L-shape) in order to provide the same effect as the cutaway. For instance, the spray head may comprise a short substantially vertical section for engaging the free end of the outlet stem and have a short substantially horizontal section for directing the spray of fluid through the aperture of the device, and the spray head may have a transverse longer section between said short sections. The result of this unusual configuration of the spray head may result in the same effect as the cutaway, such that the detection means' scan of the spray head detects the difference in the magnetism of the spray head against the absence of any magnetism above the short substantially vertical section.

The areas of differing magnetism on the spray head may be provided in the form of at least one line of differing magnetism. Said one or more lines preferably being substantially perpendicular relative to an inlet section of the spray head; in other words, in a substantially horizontal direction when the refill is placed on a flat surface. Such orientation of the lines may be advantageous since it could improve the likelihood that the detection means could distinguish between lines having differing magnetism, particularly where said detection means are operable during the movement of the spray head.

The areas of differing magnetism on the spray head may be provided in the form of two lines of differing magnetism. Alternatively, the areas of differing magnetism on the spray head may be provided in the form of a plurality of lines of differing magnetism. The use of two or a plurality of lines may be advantageous as these may be able to operate as a code capable of imparting additional information to the device beyond whether the refill is a safe refill or not.

Alternatively the areas of differing magnetism could be provided in the form of one or more patterns and/or one or more shapes and/or one or more letters and/or one or more numerals and/or magnetic code.

Such further information could include the particular type of refill to permit the device to alter its mode of operation. For instance, where the refill is an insecticide, the spraying frequency may desirably be different to when the device is spraying an air freshener or the like.

As another example, the further information could relate to a specific end of life period such that the device may be operable to cease activation after a certain number of activations that corresponds to the quantity of fluid in the refill. In this arrangement the battery life of the device would be prolonged as the device would no longer activate once the certain number of activations had been reached. Once the device had reached a certain number of activations, the device would remain dormant until a user replaced the refill and reset the device.

Alternatively, areas of magnetism could impart said further information wherein the detection means is adapted to recognize the presence of a specific magnetic property, said specific magnetic property may be referenced against an internal memory of such properties in order to permit the device to recognize the further information.

The device of any of the above-mentioned aspects may be provided with an indicator wherein said indicator is operable to indicate information to a user. Such information may include: whether the refill loaded in the device is potentially dangerous as it is not intended for use with the device; whether the refill needs to be changed; whether the battery or batteries need to be changed (where the device is to be battery powered); and other potentially useful information.

The indicator may be operable to provide a visual indication and/or provide an audible indication.

Preferably the indicator is configured to provide a visual indication by emitting light from one or more light sources, preferably one or more LEDs. The one or more light sources may be adapted to emit a different colour of light to indicate the current function the device is performing. Additionally or alternatively, the one or more light sources may blink or flash to indicate the current function the device is performing.

A visual indicator may be provided in the form of an LCD screen or the like wherein the screen is adapted to provide a message to a user, for instance such messages could include "ON", "DANGEROUS REFILL INSERTED", "CHANGE REFILL", "CHANGE BATTERIES", "NUMBER OF SPRAYS REMAINING", "LIFE OF REFILL", "OFF".

The device may be provided with a boost mechanism. The boost mechanism may be linked to a user operated switch or button or the like. On operating the boost mechanism the actuation means may activate to cause the immediate actuation of a refill.

The device may be power by mains-supplied electricity and/or be battery powered and/or be powered by solar cells located on the device. Most preferably the device is battery powered.

According to a sixth aspect of the present invention there is provided therefore a spray head for a refill for use with a device according to any of the first to fourth aspects of the present invention and for use in the method according to either of the fifth or sixth aspects of the present invention wherein the spray head comprises an inlet section adapted to engage with a refill body and an outlet section suitable for directing a spray of fluid away from the refill body, wherein the spray head has an external surface having at least one area of lower magnetism and at least one area of relatively higher magnetism.

According to a seventh aspect of the present invention there is provided therefore a refill for use with a device according to any of the first to fourth aspects of the present invention and for use In the method according to either of the fifth or sixth aspects of the present invention wherein the refill comprises a body for containing a quantity of fluid and a spray head, said spray head comprising an inlet section adapted to engage with a refill body and an outlet section suitable for directing a spray of fluid away from the refill body, wherein the spray head has an external surface having at least one area of lower magnetism and at least one area of relatively higher magnetism.

According to a eighth aspect of the present invention there is provided therefore a kit of parts for spraying a quantity of fluid, said kit comprising a device in accordance with any of the first to fourth aspects of the present invention, said device adapted to operate in accordance with a method according to either of the fifth or sixth aspects of the present invention, the kit further comprising a refill in accordance with the seventh aspect of the present invention.

### Brief Description of the Drawings

Embodiments of the invention will now be described, by way of example only, with reference to the following drawings in which:
Fig. 1 illustrates a side elevation of a device of the present invention with a refill loaded therein;
Fig. 2 illustrates a front elevation of a device of the present invention with a refill loaded therein; and
Fig. 3 illustrates a perspective view of a spray head for a refill.

### Description of an Embodiment

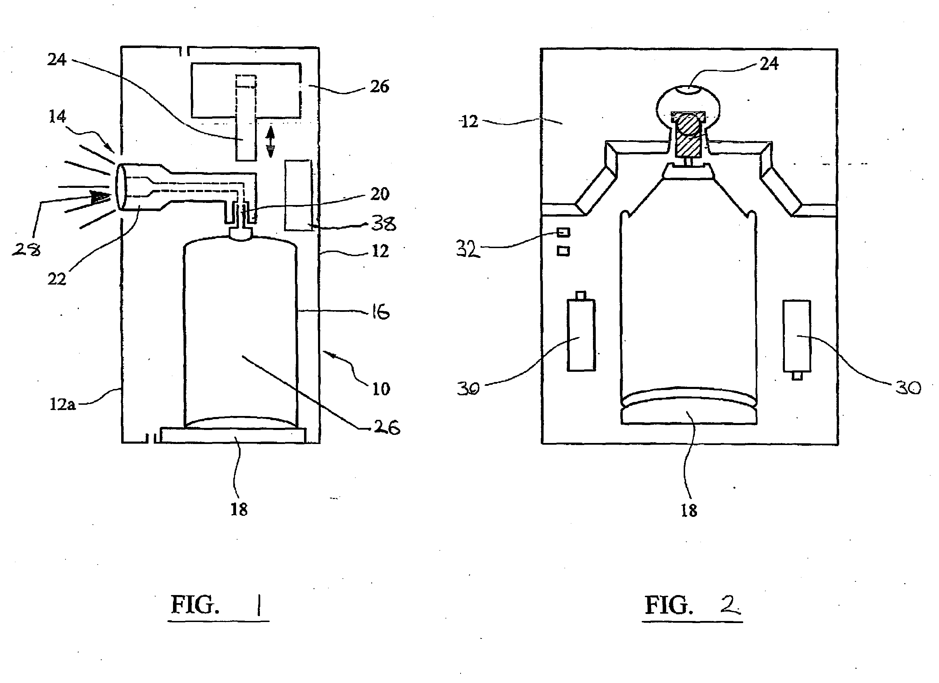

As shown in Figures 1 and 2 a fragrance spraying device 10 comprises a housing 12 with a removable front section 12a of which there is an opening 14. Alternatively, the front section 12a may be hinged to permit access to the interior of the device 10. A refill 16, in this example an aerosol spray canister, is held within the housing 12 on a platform 18. An outlet stem 20 of the spray canister 16 is received in a lower opening in a spray head 22. Actuation means 24 is located above the refill 16 and possesses an arm 24 that is moveable to apply substantially downward pressure on the spray head 22 and cause activation of the refill 16. During activation of the refill, fluid held within the body 26 of the refill 16 is forced through the outlet stem 20, into the spray head, exiting the spray head via a outlet in the form of a nozzle 28 into the external environment. The actuation means 26 are powered by batteries 30.

The device is further provided with a detection means 38 located substantially adjacent to the location of the spray head 22 when the refill is loaded into the device 10.

Although not shown, the detection means 38 includes a sensor configured to face the spray head 22, the sensor being adapted to detect and distinguish between different areas of magnetism on the spray head 22.

The detection means 38 can be in direct communication with the actuation means 26 or, alternatively, in communication with a control unit (not shown) which is in communication with the actuation means 26. The communication between the detection means 38 and the actuation means 26, whether directly or indirectly via the control unit, being essential to permit the device to be capable of producing a response when a dangerous and/or abusive refill is loaded into the device 10.

In use, the refill 16 is placed on the platform 18 and the outlet stem 20 is engaged in an inlet 34 of the spray head 22. The refill 16 is a replaceable item, whilst the spray head 22 is typically supplied with the refill 16.

When the refill 16 is placed in position a fluid path for fragrance (or other material, such as sterilising material/insecticide material/bactericide material or the like) for spraying is formed from the refill 16 through the spray head 22 to the opening 14 in the front section of the housing 12a and out into the surrounding environment.

In order to cause spraying of the material within the refill 16, the arm 24 is caused to move down onto the spray head 22. The actuation means 26 has numerous selectable settings which a user may select via a user input means 32. The user input means 32 can be operable to allow a user to select whether the device is turned on or off, the specific operation mode of the device such as its spray frequency, timer delay or other such feature. Preferred frequency settings would be the option of the fluid being sprayed from the refill 16 every nine minutes, or every eighteen minutes, or every thirty-six minutes.

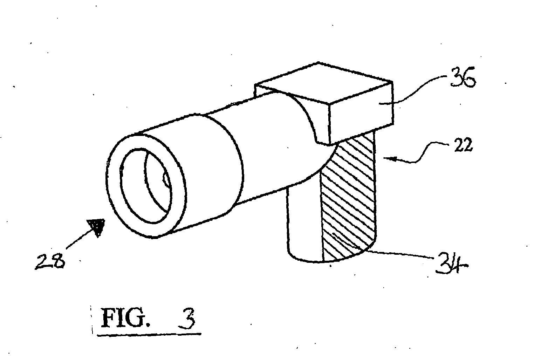

Referring to Fig. 3, the areas of differing magnetism on the spray head 22 can be seen. Specifically, the neck 34 of the spray head 22 is coated with a layer of dark ink/paint/lacquer which is of a lower magnetism than the uncoated crown 36 of the spray head 22.

In one embodiment of the invention, the detection means can be operable to detect the presence of the spray head 22 and detect whether there is at least one area of low magnetism and at least one area of higher magnetism on the spray head 22. To achieve this detection, the detection means 38 may be configured to interrogate the spray head 22 of the refill, or the area normally occupied by said spray head. The detection means 38 may be capable of attributing specifically or approximately or generally the detected magnetism to a section or sections of the spray head 22, or area normally occupied thereby. Such attribution may permit the detection means 38 to directly, or in combination with the control unit (not shown), determine whether any areas of differing magentism are present on the spray head 22 in the area normally occupied thereby.

Alternatively, the interrogation of the refill by the detection means 38 may detect the level or levels of magnetism present without attributing the level(s) to a specific section of the spray head or the area normally occupied thereby. Any such detection may permit the detection means 38 to directly, or in combination with the control unit (not shown), determine whether any areas of differing magnetism are present on the spray head or in the area normally occupied thereby.

With either form of interrogation, if differing areas of magnetism, if such differing areas are detected the detection means 38 will communicate with the actuation means 26, either directly or indirectly, to permit the actuation means 26 to operate in accordance with the user Input command to cause the spraying of fluid.

Conversely, if differing areas of magnetism are not detected, i.e. a potentially dangerous refill has been loaded into the device, the direct or indirect communication from the detection means 38 will call for the actuation means 26 to enter a dormant mode. During the dormant mode the actuation means 26 will not activate and not cause the spraying of fluid. The dormant mode will be maintained until a user initiates a resetting of the device. The resetting may be facilitated by the user loading a new refill into the device and/or the user operating a reset button or the like. However, if a vandal has intentionally loaded a potentially dangerous refill, once the detection means 38 scans the spray head 22 and fails to find the required magnetism, the actuation means 26 will again be placed in the dormant mode.

In an alternative embodiment, the detection means can be operable to detect the presence of the spray head 22 and detect whether there is at least one area of low magnetism and at least one area of higher magnetism on the spray head 22 only during the movement of the spray head 22 following activation by the actuation means 26. This movement permits the detection means 38 the opportunity to view a greater proportion of the spray head 22. If during the scanning of the spray head 22 differing areas of magnetism are detected by the detection means 38, these means will communicate with the actuation means 26, either directly or indirectly, to permit the actuation means 26 to operate in accordance with the user input command to cause the spraying of fluid.

Conversely, if differing areas of magnetism are not detected, i.e. a potentially dangerous refill has been loaded into the device, potentially to effect vandalism, the communication from the detection means 38 will call for the actuation means 26 to enter a dormant mode, as discussed above.

When a refill 16 has been loaded in the device 10 and the detection means 38 has been able to distinguish between one area of lower magnetism and once area of higher magnetism, the control means or the like may initiate a counting mechanism. The counting mechanism may be calibrated to permit a pre-determined number of actuations of the refill which corresponds to the quantity of fluid stored in a refill. The counting mechanism can be operable in use, and after the pre-determined number of actuations has been reached, to cause the device 10 to enter the dormant mode, thus prevent further actuations of that refill, until a user replaces the refill and/or resets the device. The counting mechanism may automatically reset every time a refill is loaded into the device.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A spraying device comprising a housing adapted to receive a refill of fluid therein and having an aperture suitable for permitting, in use, the spraying of a fluid therethrough, the device further comprising actuation means configured for periodic actuation of the refill, wherein the device is provided with detection means configured to distinguish between at least one area of lower magnetism and at least one area of relatively higher magnetism on a spray head of said refill or within the area normally occupied by said spray head.

2. A spraying device comprising a housing adapted to receive a refill of fluid therein and having an aperture suitable for permitting, in use, the spraying of a fluid therethrough, the device further comprising actuation means configured for periodic actuation of the refill, wherein the device is provided with detection means configured to distinguish between at least one area of lower magnetism and at least one area of relatively higher magnetism on a spray head of said refill or within the area normally occupied by said spray head, and wherein said detection means operate during movement of the spray head.

3. A spraying device comprising a housing adapted to receive a refill of fluid therein and having an aperture suitable for permitting, in use, the spraying of a fluid therethrough, the device further comprising actuation means configured for periodic actuation of the refill, wherein the device is provided with detection means configured to distinguish between one area of lower magnetism and one area of relatively higher magnetism on a spray head of said refill or within the area normally occupied by said spray head.

4. A spraying device comprising a housing adapted to receive a refill of fluid therein and having an aperture suitable for permitting, in use, the spraying of a fluid therethrough, the device further comprising actuation means configured for periodic actuation of the refill, wherein the device is provided with detection means configured to distinguish between one area of lower magnetism and one area of relatively higher magnetism on a spray head of said refill or within the area normally occupied by said spray head, and wherein said detection means operate during movement of the spray head.

5. A spraying device according to claim 2 or claim 4, wherein the detection means are configured to only be operable during the movement of the spray head.

6. A spray device according to any of claims 2, 4-5, wherein the detection means are only operable during the substantially upward movement of the spray head.

7. A spray device according to any preceding claim, wherein the device incorporates a counting mechanism to allow, in use, a pre-defined number of actuations of a refill.

8. A method of spraying a fluid from a device, the method comprising the steps of loading a refill in a spray device according to any preceding claim, placing the device in an operation mode, said mode being configured to cause activation of actuation means, said actuation means bearing against a spray head of the refill and causing the movement thereof to release a quantity of fluid from the refill, said released fluid being sprayed from the device through an aperture into the surrounding environment, wherein detection means provided in the device are operable in the operation mode to detect and distinguish between at least one area of lower magnetism and at least one area of relatively higher magnetism on a spray head of said refill.

9. The method according to claim 8 wherein device is operable to prevent the activation of the activation means if the detection means is unable to detect and distinguish between said two areas of differing magnetism.

10. The method according to claim 8 wherein the detection means is configured to only be operable during the movement of the spray head.

11. The method according to any of claims 8-10, wherein the detection means is configured to only be operable during the substantially upward movement of the spray head.

12. A spray head for a refill for use with a device according to any of claims 1-7 and for use in the method according to any of claims 8-11 wherein the spray head comprises an inlet section adapted to engage with a refill body and an outlet section suitable for directing a spray of fluid away from the refill body, wherein the spray head has an external surface having at least one area of lower magnetism and at least one area of relatively higher magnetism.

13. A refill for use with a device or method according to any of claims 1-11, wherein the refill comprises a body for containing a quantity of fluid and a spray head, said spray head comprising an inlet section adapted to engage with a refill body and an outlet section suitable for directing a spray of fluid away from the refill body, wherein the spray head has an external surface having at least one area of lower magnetism and at least one area of relatively higher magnetism.

14. The device, method, spray head or refill according to any preceding claim wherein, the areas of differing magnetism on the spray head is provided in the form of one line of differing magnetism.

15. A kit of parts for spraying a quantity of fluid, said kit comprising a device in accordance with any preceding device claim, said device adapted to operate in accordance with a method according to any preceding method claim, the kit further comprising a refill in accordance with any preceding refill claim.
